# EUROPEAN PATENT APPLICATION

(11) **EP 4 529 903 A1**
(43) Date of publication of application: **02.04.2025**
(21) Application number: 23835078.9
(22) Date of filing: 06.01.2023
(51) Int. Cl.: A61F 9/007

(54) **INTRAOCULAR DRUG ADMINISTRATION DEVICE, AGENT RESERVOIR, AND METHOD FOR OPERATING INTRAOCULAR DRUG ADMINISTRATION DEVICE**

(30) Priority: 08.07.2022 JP 2022110816
(71) Applicant: Terumo Kabushiki Kaisha, Tokyo 151-0072 (JP)
(72) Inventor: SHIMAMURA, Yoshihisa, Ashigarakami-gun, Kanagawa 259-0151 (JP); TANAKA, Ryo, Ashigarakami-gun, Kanagawa 259-0151 (JP); MASAKI, Kenji, Ashigarakami-gun, Kanagawa 259-0151 (JP); IWASE, Yoichiro, Ashigarakami-gun, Kanagawa 259-0151 (JP)
(74) Representative: Casalonga
(86) International application number: PCT/JP2023/000229
(87) International publication number: WO 2024/009535

(57) **Abstract**

An intraocular drug administration device comprising: an agent reservoir which has an outer cylindrical part that is attached to an eyeball of an organism and that retains a drug solution in a state of being in contact with the eyeball; a first electrode which is provided to the outer cylindrical part so as to be spaced away from the eyeball and to cover at least a portion of the eyeball and which is bent so as to be recessed in a direction away from the eyeball; a second electrode which is attached to the eyeball of the organism or to a site other than the eyeball; and a power supply that supplies electric current between the first electrode and the second electrode.

## Description

### TECHNICAL FIELD

The present invention relates to an intraocular drug administration device that uses an electrical driving force to administer a substance into an eye, an agent reservoir, and a method for operating the intraocular drug administration device.

### BACKGROUND ART

Ocular diseases including cornea, lens, and vitreous body are treated by medication. Eye drops are generally used as a method for administering a drug for ocular diseases. In recent years, various polymer medicines including nucleic acid medicines, which are considered to be highly effective against ocular diseases, have been proposed. However, administration of a polymer medicine by eye drops has a problem that a drug migration rate to a target site is low.

As a method for administering a drug instead of eye drops, there are corneal injection, vitreous injection, drug modification, and an administration method using an electrical driving force. Documents related to an administration method using an electrical driving force include, for example, Japanese Patent No. 4308353, U.S. Patent No. 8838229, and "Electrically assisted delivery of macromolecules into the corneal epithelium", Jinsong Hao et al., Exp Eye Res. 2009 December; 89(6): 934-941.

### SUMMARY OF THE INVENTION

Corneal injection is a method for puncturing a cornea with an injection needle to administer a drug, and is excellent in the migration ability of drug, but is highly invasive, and is considered to have a problem in safety. In addition, the vitreous injection enables puncture with an injection needle to a site avoiding the corneal endothelium, but there is a problem that the migration ability of drug is low in a case where the target site of drug administration is a tissue other than the vitreous body such as the cornea.

The drug modification is a technique for improving drug permeability by emulsifying a drug or the like, and can be administered by eye drops. However, the improvement of the drug migration rate by drug modification remains about several times, and sufficient migration ability cannot be obtained.

The administration method using an electrical driving force is a method for migrating a drug having a charge into the eye in a state where an electric field is applied to the eyeball. However, in the conventional administration device, the migration ability of drug tends to decrease in proportion to the molecular weight, and sufficient migration ability is not obtained for a polymer medicine such as a nucleic acid medicine.

An object of the present invention is to solve the problem described above.

One aspect of the following disclosure is (1) an intraocular drug administration device including: an agent reservoir that is attached to an eyeball of an organism, and has an outer cylinder portion holding an agent in a state of being in contact with the eyeball; a first electrode that is provided in the outer cylinder portion so as to be separated from the eyeball and cover at least a part of the eyeball, and has a curved shape so as to be recessed in a direction away from the eyeball; a second electrode that is attached to an organism site energizable between the second electrode and the first electrode; and a power supply that supplies a current between the first electrode and the second electrode.

The intraocular drug administration device and the agent reservoir from the above viewpoint can apply a uniform electric field to the surface of the tissue of the eyeball (for example, cornea or the like) through the first electrode. Therefore, it is possible to efficiently migrate the drug to the inside of the tissue using the wide area of the surface of the tissue of the eyeball and to increase the amount of the drug migrated to the tissue of the eyeball.

(2) In the intraocular drug administration device according to (1), the first electrode may have a curved surface shape. This intraocular drug administration device can apply a uniform electric field to the corneal surface and can migrate more drug into the cornea.

(3) In the intraocular drug administration device according to (2), the curved surface shape of the first electrode is a shape along a spherical surface or an elliptical surface.

(4) In the intraocular drug administration device according to any one of (1) to (3), the first electrode may have a curved shape with a curvature radius of 6 mm to 20 mm. In such an intraocular drug administration device, since the first electrode is disposed at a substantially constant distance with respect to the substantially entire region of the cornea, a uniform electric field can be applied to the corneal surface.

(5) In the intraocular drug administration device according to any one of (1) to (4), the second electrode is attached to the same eyeball as the eyeball to which the agent reservoir is attached, and a lower end portion of the outer cylinder portion has a circular contact portion surrounding the corneal limbus of the eyeball, and the second electrode is disposed continuously or dispersedly so as to surround a cornea along an annular region surrounding the contact portion. In this intraocular drug administration device, the electric conduction range can be limited only to the anterior segment.

(6) In the intraocular drug administration device according to (5), the second electrode may be attached to a conjunctiva. This intraocular drug administration device can suppress damage to the posterior ocular tissues.

(7) In the intraocular drug administration device according to (5) or (6), the second electrode may be electrically connected to the eyeball via a conductive covering layer. Since the second electrode is not in direct contact, damage to the tissue of the eye surface can be suppressed.

(8) In the intraocular drug administration device according to any one of (1) to (7), the second electrode may be attached to a forehead of an organism.

(9) In the intraocular drug administration device according to any one of (1) to (8), an inner surface of the outer cylinder portion of the agent reservoir may have a radial clearance outside a corneal limbus of the eyeball. Since the intraocular drug administration device can apply an electric field to the convex cornea so as to wrap from the outside, a uniform electric field can be applied over a wide range of the corneal surface.

(10) In the intraocular drug administration device according to any one of (1) to (9), the outer cylinder portion of the agent reservoir may have an inner diameter larger than a diameter of a cornea of the eyeball. Since the intraocular drug administration device can apply an electric field to the convex cornea so as to wrap from the outside, a uniform electric field can be applied over a wide range of the corneal surface. As a result, the intraocular drug administration device can apply a uniform electric field to substantially the entire region of the corneal surface.

(11) In the intraocular drug administration device according to (9) or (10), the outer cylinder portion of the agent reservoir may have a reduced diameter portion whose inner diameter decreases as approaching the eyeball between the first electrode and the eyeball. This intraocular drug administration device can suppress a decrease in the electric field near the corneal limbus and apply a uniform electric field to the entire cornea.

(12) In the intraocular drug administration device according to any one of (9) to (11), the first electrode may have an outer diameter equal to or larger than a diameter of the corneal limbus. This intraocular drug administration device can suppress a decrease in the electric field near the corneal limbus and apply a uniform electric field to the entire cornea.

(13) In the intraocular drug administration device according to (12), the first electrode may have a diameter of 9.1 mm to 30 mm. The first electrode can apply a uniform electric field to substantially the entire region of the corneal surface.

(14) In the intraocular drug administration device according to (12), an outer diameter of the first electrode may be smaller than an inner diameter of an upper end of the reduced diameter portion, and have a size equal to or larger than an inner diameter of a lower end of the reduced diameter portion. The first electrode can apply a uniform electric field to substantially the entire region of the corneal surface.

(15) In the intraocular drug administration device according to any one of (1) to (14), the first electrode may have a through-hole through which a fluid passes. The intraocular drug administration device can inject the drug solution into the gap between the first electrode and the cornea or discharge air without being hindered by the first electrode, so that handleability is improved.

(16) In the intraocular drug administration device according to (15), the through-hole may be formed in a central portion of the first electrode. In this intraocular drug administration device, when the agent reservoir is disposed on the eyeball, the through-hole is disposed at the central portion located at the highest position in the first electrode. As a result, since the intraocular drug administration device can inject the drug solution without leaving air in the gap between the first electrode and the cornea, it is possible to suppress the variation in the electric field.

(17) In the intraocular drug administration device according to any one of (1) to (16), the agent reservoir may have a seal member made of an elastic member at a lower end portion. This intraocular drug administration device can prevent leakage of the drug solution from the agent reservoir.

(18) The intraocular drug administration device according to any one of (1) to (17), further including: a control device that controls the power supply; and a storage unit that stores a command to be executed by the control device, in which when the command is executed, the control device may perform a first step of applying a high-voltage pulse between the first electrode and the second electrode through the power supply. According to this intraocular drug administration device, it is possible to efficiently migrate a drug having a high molecular weight into the cornea by forming a local transport region on the corneal surface in the first step.

(19) In the intraocular drug administration device according to (18), the control device may perform a second step of causing a constant current having a smaller current value than the high-voltage pulse to flow between the first electrode and the second electrode through the power supply for a predetermined time after the first step. Since the intraocular drug administration device can efficiently migrate the drug into the cornea through the local transport region in the second step, the migration rate of the drug can be further increased.

(20) Another aspect is an agent reservoir, including: an outer cylinder portion that is attached to an eyeball of an organism and holds an agent in a state of being in contact with the eyeball; and a first electrode that is provided in the outer cylinder portion to be separated from the eyeball to cover at least a part of the eyeball, and is curved to be recessed in a direction away from the eyeball.

(21) Still another aspect is a method for operating an intraocular drug administration device including an agent reservoir that is attached to an eyeball of an organism and has an outer cylinder portion holding an agent in a state of being in contact with the eyeball, a first electrode that is provided in the outer cylinder portion so as to be separated from the eyeball and cover at least a part of the eyeball, and is curved so as to be recessed in a direction away from the eyeball, a second electrode that can be energized between the first electrode and the second electrode, a power supply that supplies a current between the first electrode and the second electrode, a control device that controls the power supply, and a storage unit that stores a command to be executed by the control device, the method including: a first step, performed by the control device, of controlling the power supply to apply a high-voltage pulse between the first electrode and the second electrode a plurality of times.

In the method for operating the intraocular drug administration device from the above viewpoint, it is possible to efficiently migrate a drug having a high molecular weight into the cornea by forming a local transport region on the corneal surface in the first step.

(22) In the method for operating the intraocular drug administration device according to (21), the method may include: a second step of causing a constant current having a smaller current value than the high-voltage pulse to flow between the first electrode and the second electrode through the power supply for a predetermined time after the first step. This operation method can further increase the migration rate of drug.

### BRIEF DESCRIPTION OF DRAWINGS

[FIG. 1] Fig. 1 is a configuration diagram of an intraocular drug administration device according to a first embodiment.
[FIG. 2] Fig. 2A is a cross-sectional view of an agent reservoir of Fig. 1, and Fig. 2B is a cross-sectional view of a first electrode taken along line IIB-IIB of Fig. 2A.
[FIG. 3] Fig. 3A is a flowchart illustrating a first administration method using the intraocular drug administration device of Fig. 1, and Fig. 3B is a flowchart illustrating a second administration method using the intraocular medicine administration device of Fig. 1.
[FIG. 4] Fig. 4 is an explanatory view of a step of injecting a drug solution into the agent reservoir of Fig. 2A.
[FIG. 5] Fig. 5 is a schematic cross-sectional view (left) of the cornea before voltage application, a schematic cross-sectional view (center) of the cornea immediately after application of a high-voltage pulse, and a schematic cross-sectional view (right) of the cornea in which a constant current is applied.
[FIG. 6] Fig. 6A is a plan view of a first electrode according to a first modification of the first embodiment, and Fig. 6B is a plan view of a second electrode according to a second modification of the first embodiment.
[FIG. 7] Fig. 7A is a cross-sectional view of an agent reservoir according to a third modification of the first embodiment, and Fig. 7B is a cross-sectional view of an agent reservoir according to a fourth modification of the embodiment.
[FIG. 8] Fig. 8A is a cross-sectional view of an agent reservoir according to a fifth modification of the first embodiment, and Fig. 8B is a cross-sectional view of an agent reservoir according to a sixth modification of the first embodiment.
[FIG. 9] Fig. 9A is a cross-sectional view of an agent reservoir according to a seventh modification of the first embodiment, and Fig. 9B is a cross-sectional view of an agent reservoir according to an eighth modification of the first embodiment.
[FIG. 10] Fig. 10 is a diagram (Experimental Example 1) illustrating a migration evaluation result of the fluorescence-modified nucleic acid by fluorescence imaging to a porcine cornea using a high-voltage pulse (EP), a constant current (IP), and a combination method of a high-voltage pulse and a constant current (EP+IP: embodiment) and a tissue degeneration evaluation result by an HE staining method.
[FIG. 11] Fig. 11 is a graph (Experimental Example 1) illustrating an evaluation result of the migration amount of the nucleic acid medicine into the porcine cornea when no voltage is applied (control), the high-voltage pulse (EP), the constant current (IP), and a combination of the high-voltage pulse and the constant current (EP+IP: embodiment).
[FIG. 12] Fig. 12A is a cross-sectional view of an agent reservoir and an active electrode according to Comparative Example 1, and Fig. 12B is a diagram illustrating the migration evaluation result of a fluorescence-modified nucleic acid by fluorescence imaging to a porcine cornea in the agent reservoir (Comparative Example 1) of Example 1 and Fig. 12A.
[FIG. 13] Fig. 13A is a diagram illustrating a result obtained by calculating an electric field distribution of a corneal surface using a finite element method when a high-voltage pulse is applied to an agent reservoir of Comparative Example 1, and Fig. 13B is a diagram illustrating a calculation result of an electric field distribution of a corneal surface when a high-voltage pulse is applied by an agent reservoir of Comparative Example 2.
[FIG. 14] Fig. 14A is a diagram illustrating a calculation result of an electric field distribution of the cornea surface by the administration device according to Comparative Example 3, and Fig. 14B is a diagram illustrating a calculation result of an electric field distribution of the cornea surface by the administration device according to Comparative Example 4.
[FIG. 15] Fig. 15A is a diagram illustrating a calculation result of an electric field distribution of the cornea surface by the administration device according to Example 1, and Fig. 15B is a diagram illustrating a calculation result of an electric field distribution of the cornea surface by the administration device according to Example 2.
[FIG. 16] Fig. 16A is a diagram illustrating a calculation result of an electric field distribution of the cornea surface by the administration device according to Example 3, and Fig. 16B is a diagram illustrating a calculation result of an electric field distribution of the cornea surface by the administration device according to Example 4.
[FIG. 17] Fig. 17A is a diagram illustrating a calculation result of an electric field distribution of the cornea surface by the administration device according to Example 5, and Fig. 17B is a diagram illustrating a calculation result of an electric field distribution of the cornea surface by the administration device according to Example 6.
[FIG. 18] Fig. 18 is a configuration diagram of an intraocular drug administration device according to a second embodiment.
[FIG. 19] Fig. 19A is a cross-sectional view of the agent reservoir of Fig. 18, Fig. 19B is a cross-sectional view taken along line XIXB-XIXB of Fig. 19A, and illustrates a shape of the second electrode of Fig. 19A.
[FIG. 20] Fig. 20 is an enlarged cross-sectional view of the vicinity of a second electrode of an agent reservoir according to a modification of the second embodiment.
[FIG. 21] Fig. 21A is a diagram illustrating a result obtained by calculating an electric field distribution at a corneal surface using a finite element method when a high-voltage pulse is applied to an agent reservoir of Example 7 (second embodiment), and Fig. 21B is a diagram illustrating a calculation result of an electric field distribution at a corneal surface when a high-voltage pulse is applied by the agent reservoir of Example 7.

### DESCRIPTION OF THE INVENTION

### (First Embodiment)

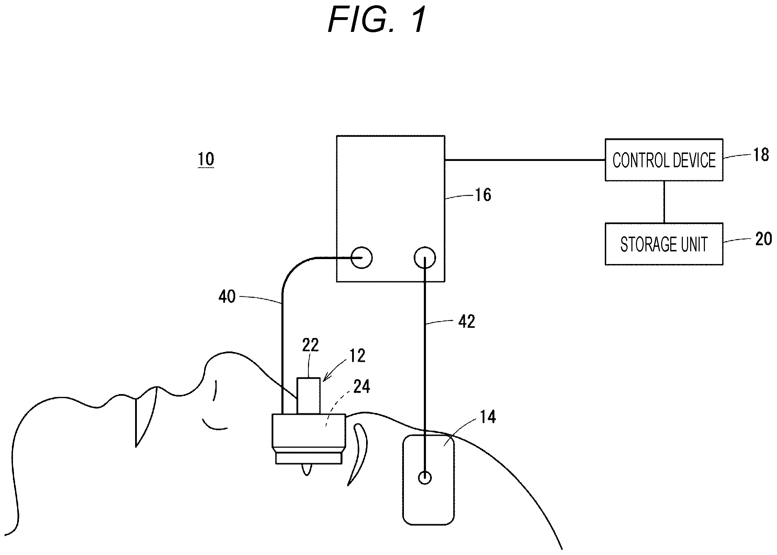

As illustrated in Fig. 1, an intraocular drug administration device 10 according to the present embodiment is used to administer a drug to the inside of a cornea 92 using an agent reservoir 12 to be attached to an eyeball 90 of a patient.

The intraocular drug administration device 10 includes an agent reservoir 12, a second electrode 14, a power supply 16, a control device 18, and a storage unit 20. The agent reservoir 12 is an instrument to be attached to the eyeball 90.

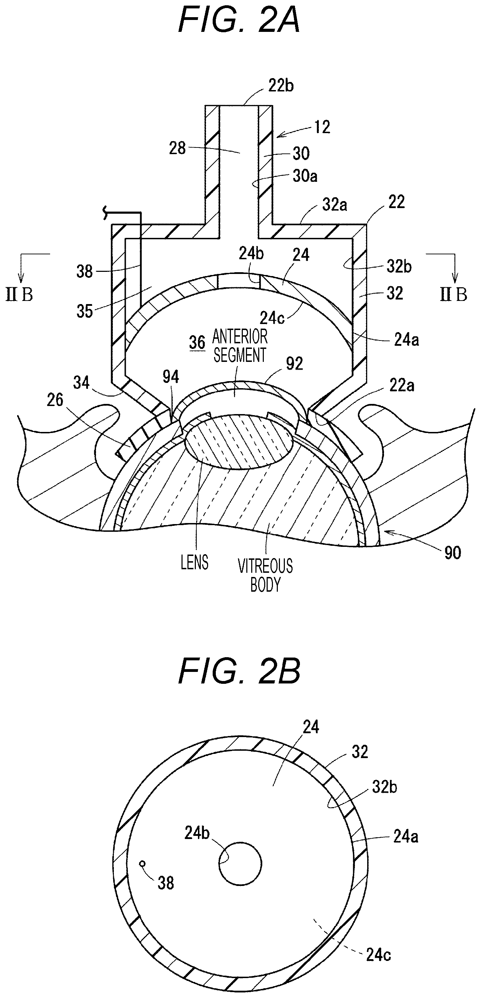

As illustrated in Fig. 2A, the agent reservoir 12 includes a main body portion 22, a first electrode 24, and a seal member 26. The main body portion 22 is a cylindrical member, and is integrally formed of, for example, a transparent resin material such as an acrylic resin (PMMA). The main body portion 22 has a cavity 28 therein. The cavity 28 accommodates a drug solution. The main body portion 22 has two openings including an opening for allowing the drug solution in the cavity 28 to flow into the cavity 28 and an opening for bringing the drug solution in the cavity into contact with the eyeball 90 (cornea 92) of the patient. Specifically, the main body portion 22 opens at a lower end portion 22a abutting on the eyeball 90 and an upper end portion 22b opposite to the lower end portion 22a. The main body portion 22 includes a port portion 30 formed on the upper end side, an outer cylinder portion 32 adjacent to the lower end side of the port portion 30, and a reduced diameter portion 34 provided at the lower end of the outer cylinder portion 32. In the present embodiment, the port portion 30 has a cylindrical shape having an outer diameter smaller than the outer diameter of the outer cylinder portion 32. However, as long as a drug solution can be injected, the port portion does not need to be a structure having a cylindrical shape or the like, and may be an opening provided in the main body portion 22. The port portion 30 has a flow path 30a through which the drug solution passes. The flow path 30a constitutes a part of the upper end side of the cavity 28.

The outer cylinder portion 32 is a cylindrical portion having an outer diameter larger than that of the port portion 30. The outer cylinder portion 32 is integrally connected to the port portion 30 via a step portion 32a. The outer cylinder portion 32 has an inner side surface 32b that defines a housing chamber 36 to be described later. The inner side surface 32b has an inner diameter larger than the diameter of the cornea 92 (corneal limbus 94) of the patient. When viewed from the axial direction of the agent reservoir 12, the inner side surface 32b forms a clearance with the corneal limbus 94. The diameter of the corneal limbus 94 increases from newborn to adult, and its width is 9 mm to 12 mm. Therefore, the inner diameter of the inner side surface 32b of the outer cylinder portion 32 can be set to, for example, 9.1 mm to 30 mm, and is preferably 9.5 mm to 20 mm. The housing chamber 36 for storing the drug solution injected from the port portion 30 and an upper space 35 are formed inside the inner side surface 32b. The housing chamber 36 constitutes a part of the cavity 28 on the lower end side of the flow path 30a. That is, the housing chamber 36 is located on the lower end portion 22a side of the first electrode 24. The upper end side of the housing chamber 36 communicates with the flow path 30a, and the lower end side opens downward. The housing chamber 36 is a space for housing a drug solution injected by a syringe or the like. The upper space 35 is located above the housing chamber 36. The upper space 35 is a portion that stores the drug solution overflowing from the housing chamber 36.

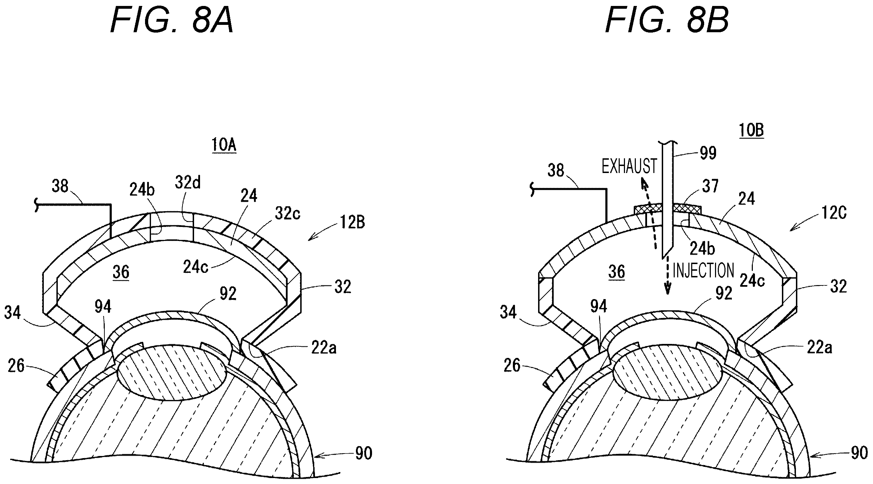

The outer cylinder portion 32 has the first electrode 24 therein. In the illustrated example, the first electrode 24 protrudes from the middle in the axial direction of the outer cylinder portion 32 toward the central axis (specifically, the central portion) of the outer cylinder portion 32, but the present embodiment is not limited thereto. The first electrode 24 may be formed so as to be in contact with the step portion 32a (see Fig. 8A). The upper end of the agent reservoir 12 may be formed by the first electrode 24 (see Fig. 8B). In these cases, only the housing chamber 36 is formed inside the outer cylinder portion 32 on the lower end portion 22a side of the first electrode 24. That is, the upper space 35 is not formed on the first electrode 24.

The first electrode 24 has an outer diameter larger than the diameter of the cornea 92 (corneal limbus 94). An outer peripheral portion 24a of the first electrode 24 is in contact with the inner side surface 32b of the outer cylinder portion 32. As illustrated in Fig. 2B, the first electrode 24 is held by the outer cylinder portion 32. As illustrated in Fig. 2A, the first electrode 24 has a curved shape along the surface shape of the cornea 92. That is, the first electrode 24 has a curved surface in which a surface 24c on the cornea 92 side is curved so as to be recessed toward the upper end portion 22b as it goes toward the radial central portion of the agent reservoir 12.

The first electrode 24 is disposed with a gap (housing chamber 36) having a substantially constant width from the surface of the cornea 92. When the agent reservoir 12 is attached to the eyeball 90, the housing chamber 36 forms a gap between the first electrode 24 and the cornea 92. Therefore, the upper end side of the housing chamber 36 is defined by the first electrode 24. The outer end of the housing chamber 36 is defined by the inner side surface 32b. Therefore, the outer diameter of the housing chamber 36 is larger than the diameter of the cornea 92 (corneal limbus 94) as with the first electrode 24 when viewed from the axial direction of the agent reservoir 12. The first electrode 24 has a shape curved along a curved surface shape such as a spherical surface or an elliptical surface having a radius equal to or larger than the curvature radius (for example, 6 mm to 10 mm) of the cornea 92, for example. In this case, the curvature radius of the first electrode 24 can be, for example, 6 mm to 20 mm. The first electrode 24 is separated from the surface of the cornea 92 at a substantially constant interval by a gap.

The first electrode 24 has a through-hole 24b. The through-hole 24b is located at a central portion which is an upper end of the first electrode 24. As illustrated in Fig. 2B, the through-hole 24b is formed in a circular shape in plan view. The through-hole 24b is provided so as not to impair the function of the first electrode 24, and serves as a path for injecting a drug solution into the housing chamber 36 below the first electrode 24 in Fig. 2A and also serves as a path for discharging air bubbles in the housing chamber 36. Since the through-hole 24b is located at the upper end of the first electrode 24, the drug solution can be filled in the housing chamber 36 without leaving air bubbles in the housing chamber 36.

The first electrode 24 is formed of, for example, a conductive metal material such as gold, platinum, a titanium alloy, or stainless steel, or a carbon material. In addition, the first electrode 24 may be configured by a substrate made of a transparent material and a transparent electrode material formed on a surface of the substrate in order to improve visibility at the time of positioning with the eyeball 90. A conductive wire 38 is connected to the upper surface of the first electrode 24. The conductive wire 38 extends through the step portion 32a to the outside of the agent reservoir 12. The arrangement of the conductive wire 38 is not limited to the illustrated example, and may extend to the upper end portion 22b along the inner surface of the agent reservoir 12. The conductive wire 38 is electrically connected to the power supply 16 via a wiring 40 as illustrated in Fig. 1.

As illustrated in Fig. 2A, the reduced diameter portion 34 extends downward from the lower end of the outer cylinder portion 32. The reduced diameter portion 34 has a conical shape in which the inner diameter and the outer diameter gradually decrease toward the lower end. The inner diameter of the lower end portion of the reduced diameter portion 34 has substantially the same size as the corneal limbus 94 which is the outer peripheral portion of the cornea 92. That is, the diameter of the reduced diameter portion 34 decreases toward the corneal limbus 94 from the lower end of the outer cylinder portion 32. The seal member 26 is joined to the lower end portion of the reduced diameter portion 34. The seal member 26 is a cylindrical member having elasticity. The seal member 26 is formed of, for example, an elastic member such as a silicone elastomer or a urethane elastomer. The seal member 26 is in close contact with the eyeball 90 and fills a gap with the lower end portion of the reduced diameter portion 34, thereby preventing leakage of the drug solution from the housing chamber 36.

As illustrated in Fig. 1, the second electrode 14 is a sheet-like electrode, and is attached to a site such as a face (forehead) of a patient, for example. The second electrode 14 is electrically connected to the power supply 16 via a wiring 42.

The power supply 16 applies a high-voltage pulse and supplies a constant current between the first electrode 24 and the second electrode 14 as described later under the control of the control device 18. The control device 18 is connected to the power supply 16 and the storage unit 20. The control device 18 reads the command stored in the storage unit 20 and drives the power supply 16.

The intraocular drug administration device 10 of the present embodiment is configured as described above. Hereinafter, a method for using the intraocular drug administration device 10 and its operation and action will be described.

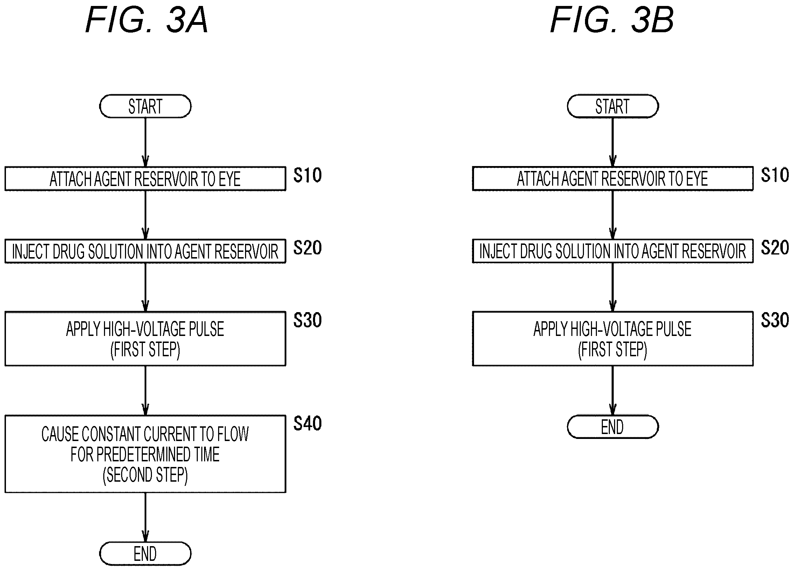

First, a first administration method using the intraocular drug administration device 10 will be described. As illustrated in Fig. 3A, the first administration method proceeds to step S10, and the agent reservoir 12 of the intraocular drug administration device 10 is attached to the eye of the patient. As illustrated in Fig. 2A, in the agent reservoir 12, the seal member 26 is brought into contact with the surface of the eyeball 90. The agent reservoir 12 is positioned such that the lower end of the reduced diameter portion 34 is aligned with the corneal limbus 94. Since the agent reservoir 12 is formed of a transparent insulating material such as resin or glass, positioning can be easily performed. In step S10, the second electrode 14 in Fig. 1 is attached to the patient's body (for example, the forehead).

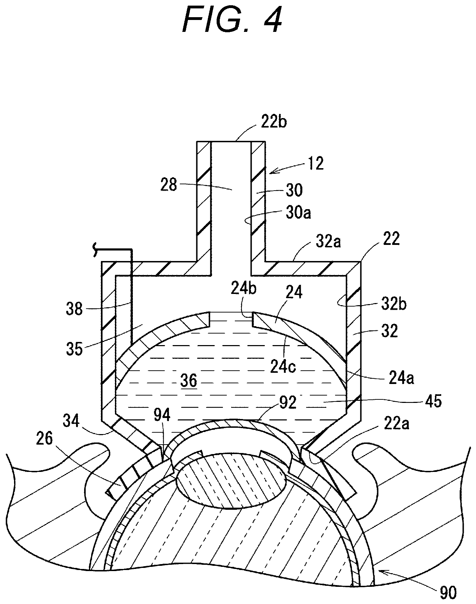

Next, the usage method proceeds to step S20 in Fig. 3A. In step S20, a drug solution (agent 45) is injected into the agent reservoir 12. As illustrated in Fig. 4, the drug solution flows into the housing chamber 36 through the flow path 30a. The drug solution is injected into the housing chamber 36 between the first electrode 24 and the cornea 92 through the through-hole 24b of the first electrode 24. The drug solution is injected until the liquid level reaches the upper end position at the center of the first electrode 24. The drug solution injected up to the upper end position can reliably cause the first electrode 24 to function. In addition, from the viewpoint of reducing the amount of valuable polymer medicine used, it is preferable to inject the drug solution only into the housing chamber 36 as illustrated in the drawing. Note that the drug solution may be injected so as to overflow from the housing chamber 36 and reach the upper space 35 above the first electrode 24 such that the entire first electrode 24 including the upper surface is immersed in the drug solution.

In the present embodiment, the drug solution includes, for example, a low molecule medicine, a nucleic acid medicine, an antibody medicine, a gene therapeutic agent, and/or a medicine such as protein.

Thereafter, the intraocular drug administration device 10 performs a first operation. The first operation includes step S30 (first step) and step S40 (second step) in Fig. 3A. The usage method proceeds to step S30 in Fig. 3A. Step S30 is a step (first step) in which the power supply 16 applies a high-voltage pulse between the first electrode 24 and the second electrode 14 under the control of the control device 18. The first step is also called electroporation.

In the present embodiment, a voltage of 1 to 500 V, preferably a voltage of 10 to 450 V, more preferably a voltage of 50 to 400 V is applied as a high-voltage pulse between the first electrode 24 and the second electrode 14. At a voltage of less than 1 V, improvement in the medicine migration amount cannot be expected. Note that the medicine migration amount increases as the voltage value of electroporation increases. The higher the voltage of the high-voltage pulse, the higher the manufacturing cost of the power supply 16 to use, thus reducing the cost effectiveness in the treatment. The upper limit value of the voltage of the high-voltage pulse is an example value set by the manufacturing cost of the power supply 16, and is not a medical restriction.

The duration of the voltage pulse may be between 0.1 ms and 1000 ms. The waveform of the high-voltage pulse is not particularly limited, and can be, for example, a rectangular wave, a sine wave, a triangular wave, a sawtooth wave, an attenuation wave, or the like. The application of the high-voltage pulse in step S30 is repeatedly performed a plurality of times. Polarities of the first electrode 24 and the second electrode 14 (directions of the positive pole and the negative pole) are set so as to generate an electric field for moving the medicine into the cornea 92. For example, in the case of a medicine with a positive charge, the power supply 16 sets the first electrode 24 to a positive pole and the second electrode 14 to a negative pole. In the case of a medicine (for example, some nucleic acid medicines) with a negative charge, the power supply 16 sets the first electrode 24 to a negative pole and the second electrode 14 to a positive pole.

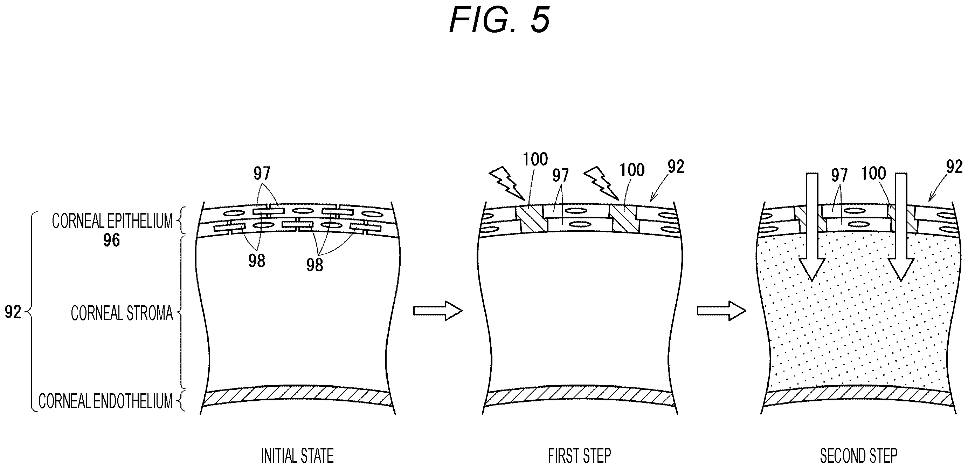

As illustrated on the left side of Fig. 5, in the initial state, a tight junction barrier 98 exists in a corneal epithelium 96 on the surface of the cornea 92. The tight junction barrier 98 is configured by a structure closely connected between cells 97. The tight junction barrier 98 prevents passage of a substance having a large molecular weight, and thus prevents migration of a polymer medicine such as a nucleic acid medicine.

When a high-voltage pulse is applied, as illustrated in the middle diagram of Fig. 5, the arrangement of the cell membrane of the cell 97 is changed, and a temporary local transport region 100 through which the polymer medicine can pass is formed in the cell membrane. The local transport region 100 is a hole penetrating the corneal epithelium 96 in the thickness direction. Typical sizes of the local transport regions 100 are in the order of 20 nm to 200 µm in diameter. A plurality of local transport regions 100 are formed on the surface of the cornea 92. When a high-voltage pulse is applied, the drug of interest (for example, a polymer medicine) is pushed from the surface of the cornea 92 to a depth of several tens µm.

The agent reservoir 12 of the present embodiment includes the first electrode 24 having a curved surface along the surface shape of the cornea 92. Since such a first electrode 24 can apply a substantially uniform electric field to the surface of the cornea 92, the local transport region 100 can be uniformly formed over the entire region of the surface of the cornea 92.

Note that the local transport region 100 formed in the first step has a temporary structure, and after a lapse of about several minutes to several hours, the arrangement of the cell membrane returns to its original state and the region disappears, and the tight junction barrier 98 also recovers.

Next, the usage method proceeds to step S40 in Fig. 3A. Step S40 is a step (second step) in which the power supply 16 causes a constant current having a current value lower than that of the high-voltage pulse to flow between the first electrode 24 and the second electrode 14 for a predetermined time under the control of the control device 18. The second step is also called iontophoresis. The second step is performed after the first step and before the tight junction barrier 98 recovers.

In the second step, the power supply 16 causes, for example, a current of 0.1 to 20 mA to flow for about several 10 seconds to several minutes. As the magnitude of the current flowing in the second step, the current density with respect to the surface area of the cornea 92 can be set to 0.1 to 5.0 mA/cm². Polarities of the first electrode 24 and the second electrode 14 (directions of the positive pole and the negative pole) are set so as to generate an electric field to move the medicine into the cornea 92. For example, in the case of a medicine (for example, some nucleic acid medicines) with a negative charge, the power supply 16 sets the first electrode 24 to a negative pole and the second electrode 14 to a positive pole.

In the second step, an electric field is generated on the surface of the cornea 92. This electric field generates an electric repulsive force in the medicine injected in the first step. As a result, as illustrated in the right diagram of Fig. 5, the medicine migrated to the local transport region 100 migrates to a deeper portion of the cornea 92. The drug solution injected into the agent reservoir 12 is in contact with the entire cornea 92, and a current uniformly flows through a contact portion of the drug solution. Thus, the second step can allow more medicine to be migrated from a large area of the cornea 92 to the interior of the cornea 92 through the local transport region 100.

Through the above steps, drug administration into the cornea 92 using the intraocular drug administration device 10 is completed.

Next, a second administration method using the intraocular drug administration device 10 will be described with reference to Fig. 3B. In the second administration method, the intraocular drug administration device 10 performs the second operation.

As illustrated in Fig. 3B, the second administration method proceeds to step S10, and the agent reservoir 12 of the intraocular drug administration device 10 is attached to the eye of the patient. Step S10 is performed similarly to the method described with reference to Fig. 3A.

Next, the second administration method in Fig. 3B proceeds to step S20, and a drug solution (agent 45) is injected into the agent reservoir 12. Step S20 is performed similarly to step S20 in Fig. 3A.

Next, the second administration method in Fig. 3B proceeds to step S30. In step S30, the intraocular drug administration device 10 performs the second operation. In the intraocular drug administration device 10, the control device 18 controls the power supply 16 to perform the first step of applying a high-voltage pulse between the first electrode 24 and the second electrode 14. The first step is electroporation, which is carried out until a sufficient amount of the agent 45 is migrated into the eye.

In the second operation, the voltage and waveform of the high-voltage pulse applied between the first electrode 24 and the second electrode 14 are similar to those in step S30 in Fig. 3A. However, the voltage is 50 V or more, preferably 100 V or more, and more preferably 150 V or more. By setting the voltage to 50 V or more, an effect of increasing the drug migration amount can be obtained only by electroporation, which is suitable. The number of repetitions may be varied (increased) such that a sufficient amount of agent 45 can be migrated.

Thus, the second operation of the intraocular drug administration device 10 is completed, and the second administration method is completed. That is, in the second operation, the intraocular drug administration device 10 performs the administration of the agent 45 only by electroporation (first step). In the intraocular drug administration device 10, the first electrode 24 has a curved shape along the surface shape of the cornea 92. Therefore, the intraocular drug administration device 10 can administer a sufficient amount of the agent 45 into the eye only by electroporation by generating a substantially uniform electric field on the surface of the cornea 92.

### (First Modification of First Embodiment)

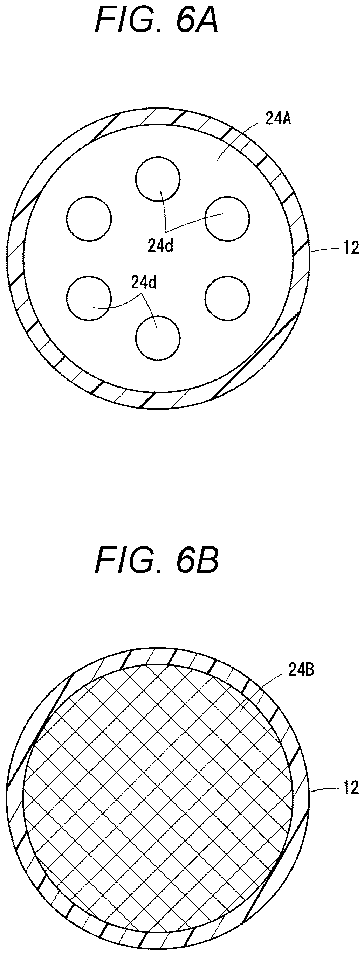

As illustrated in Fig. 6A, the intraocular drug administration device 10 of the present modification includes a first electrode 24A. The first electrode 24A of the present modification has a plurality of hole portions 24d. Each hole portion 24d can allow a drug solution to pass therethrough. The inner diameter of the hole portion 24d is formed to be sufficiently smaller than the distance between the first electrode 24A and the cornea 92, and is preferably set so as not to affect the electric field distribution formed by the first electrode 24A on the surface of the cornea 92.

### (Second Modification of First Embodiment)

As illustrated in Fig. 6B, the intraocular drug administration device 10 of the present modification includes a first electrode 24B. The first electrode 24B is formed in a mesh shape. The first electrode 24B can allow the drug solution to pass through the mesh net. The size of the mesh is formed to be sufficiently smaller than the distance between the first electrode 24B and the cornea 92, and is preferably set so as not to affect the electric field distribution formed by the first electrode 24B on the surface of the cornea 92.

### (Third Modification of First Embodiment)

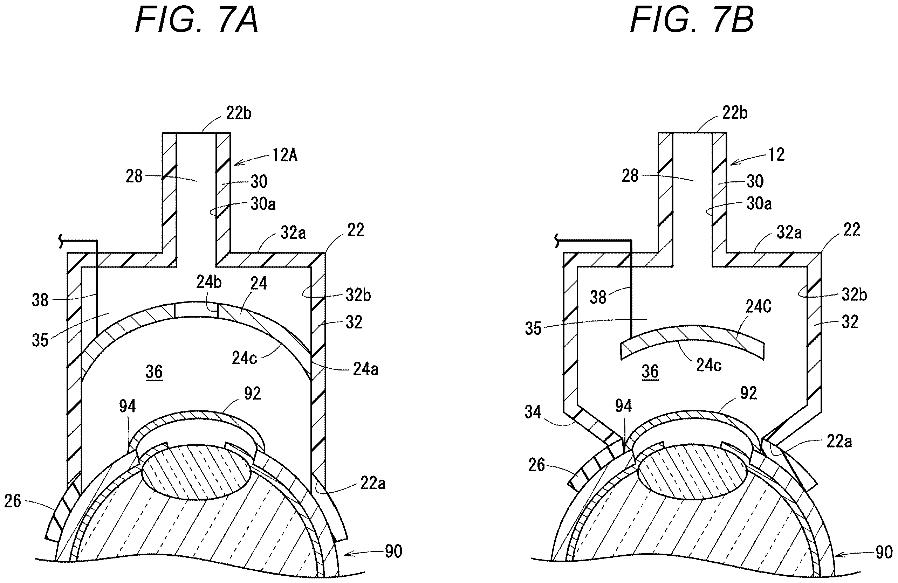

As illustrated in Fig. 7A, the intraocular drug administration device 10 of the present modification includes an agent reservoir 12A. The configuration other than the agent reservoir 12A is similar to that of the intraocular drug administration device 10 illustrated in Figs. 1 and 2A. The agent reservoir 12A of the present modification does not have the reduced diameter portion 34. That is, in the agent reservoir 12A, the outer cylinder portion 32 extends downward with a constant diameter. In the agent reservoir 12A, a seal member 26 is attached to a lower end portion of the outer cylinder portion 32. The lower end portion of the outer cylinder portion 32 abuts on the eyeball 90 via the seal member 26. According to the present modification, the same effect as that of the agent reservoir 12 of Fig. 2A can be obtained.

### (Fourth Modification of First Embodiment)

As illustrated in Fig. 7B, the intraocular drug administration device 10 of the present modification includes a first electrode 24C. The first electrode 24C is disposed instead of the first electrode 24 in Fig. 2A. The outer edge of the first electrode 24C has a gap with the inner side surface 32b of the outer cylinder portion 32, and the outer diameter thereof is set to the same value as the diameter of the cornea 92. The first electrode 24C has a curvature radius similar to that of the first electrode 24 in Fig. 2A, and has a curved shape along the cornea 92. The first electrode 24C can also apply an electric field of uniform strength to the surface of the cornea 92 in the first step, so that an effect similar to that in the case of using the first electrode 24 in Fig. 2A can be obtained, or a more excellent effect can be expected in cooperation with the gap with the inner side surface 32b.

### (Fifth Modification of First Embodiment)

The present modification relates to an agent reservoir 12B of an intraocular drug administration device 10A according to another design example of the present embodiment. As illustrated in Fig. 8A, the agent reservoir 12B includes an outer cylinder portion 32, a reduced diameter portion 34, a seal member 26, and a first electrode 24. The outer cylinder portion 32 of the present embodiment has a ceiling portion 32c at the upper end. The ceiling portion 32c has a curved shape along the first electrode 24. The first electrode 24 is joined in surface contact with the ceiling portion 32c. The first electrode 24 may be formed by bonding a conductor to the surface of the ceiling portion 32c, or may be directly formed on the surface of the ceiling portion 32c by film forming processing, plating processing, or the like.

The ceiling portion 32c has a circulation hole 32d at the center. The circulation hole 32d penetrates the ceiling portion 32c in the thickness direction. The circulation hole 32d communicates with the through-hole 24b of the first electrode 24, and communicates with the housing chamber 36 of the agent reservoir 12B via the through-hole 24b. The drug solution is injected into the housing chamber 36 through the circulation hole 32d. In addition, the circulation hole 32d enables air inside the housing chamber 36 to be discharged when the drug solution is injected into the housing chamber 36.

### (Sixth Modification of First Embodiment)

The present modification relates to an agent reservoir 12C of an intraocular drug administration device 10B according to another design example of the present embodiment. As illustrated in Fig. 8B, the agent reservoir 12C is different from the agent reservoir 12B according to the fifth modification of Fig. 8A in the first electrode 24. The first electrode 24 of the present modification also serves as the ceiling portion 32c of the outer cylinder portion 32 of Fig. 8A. The peripheral edge of the first electrode 24 is liquid-tightly joined to the upper end of the outer cylinder portion 32. The first electrode 24 has a through-hole 24b at the central portion.

The through-hole 24b is closed by a hydrophobic filter 37. The hydrophobic filter 37 is a nonwoven fabric sheet or a hydrophobic porous material made of hydrophobic fibers, and is a filter material that blocks the passage of liquid and allows the flow of gas. As illustrated in the drawing, the through-hole 24b having the hydrophobic filter 37 is used for injecting the drug solution into the housing chamber 36. The drug solution is injected by puncturing a needle tube 99 capable of penetrating the hydrophobic filter 37. When the drug solution is injected, the hydrophobic filter 37 allows passage of air and promotes exhaust inside the housing chamber 36. When the housing chamber 36 is filled with the drug solution, the hydrophobic filter 37 prevents further injection of the drug solution.

### (Seventh Modification of First Embodiment)

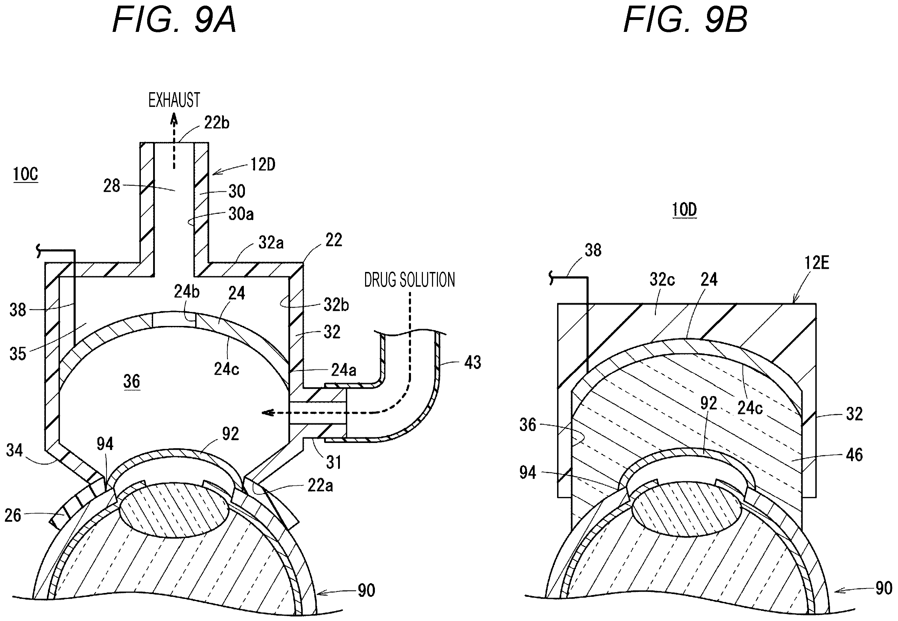

The present modification relates to an agent reservoir 12D of an intraocular drug administration device 10C according to another design example of the present embodiment. As illustrated in Fig. 9A, the agent reservoir 12D is similar to the agent reservoir 12 in Fig. 2A except that a second port portion 31 is added. The second port portion 31 is provided in the outer cylinder portion 32 on the lower end portion 22a side of the first electrode 24. The second port portion 31 communicates with the housing chamber 36 between the first electrode 24 and the eyeball 90. For example, a tube 43 is connected to the second port portion 31. The injection of the drug solution into the agent reservoir 12D is performed through the tube 43. Air in the housing chamber 36 is discharged in accordance with the injection of the drug solution through the through-hole 24b of the first electrode 24 and the flow path 30a of the port portion 30.

### (Eighth Modification of First Embodiment)

The present modification relates to an agent reservoir 12E of an intraocular drug administration device 10D according to another design example of the present embodiment. As illustrated in Fig. 9B, the agent reservoir 12E includes an outer cylinder portion 32, a ceiling portion 32c, a first electrode 24, a housing chamber 36, and an agent 46. The upper end of the outer cylinder portion 32 is closed by the ceiling portion 32c. A curved first electrode 24 is attached to the lower surface of the ceiling portion 32c. The first electrode 24 may not have the through-hole 24b (see Fig. 2A).

The housing chamber 36 is opened only on the lower end side. An upper end of the housing chamber 36 is closed by the ceiling portion 32c. The agent 46 is fitted and accommodated in the housing chamber 36. The agent 46 is a gel-like or semisolid agent containing a drug solution. The agent 46 is formed of, for example, a urethane foam impregnated with a drug solution or a hydrogel impregnated with a drug solution. The agent 46 is deformed so as to flexibly follow the shapes of the first electrode 24 and the surface of the eyeball 90. Therefore, by pressing the agent reservoir 12E against the eyeball 90 with an appropriate force, the agent 46 adheres to the front surface of the first electrode 24 and the eyeball 90 (including the cornea 92) without leaving air bubbles.

Note that the agent 46 may be disposed in the housing chamber 36 without containing the drug solution, and may be used so as to be impregnated with the drug solution after being attached to the eyeball 90.

### (Experimental Example 1)

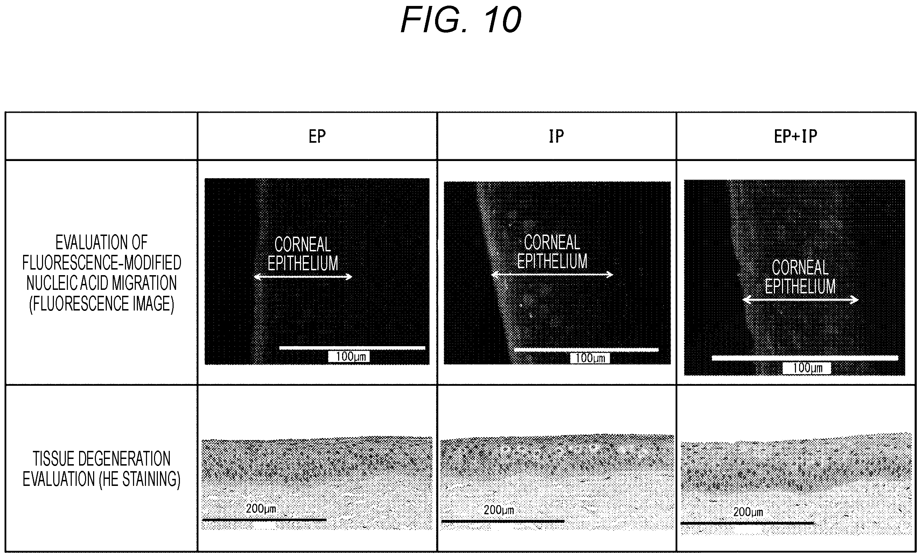

In Experimental Example 1, using the intraocular drug administration device 10 in Figs. 1 and 2A, evaluation of the migration ability of nucleic acid medicine to a porcine eyeball in vitro and evaluation of degeneration of a corneal tissue have been performed. The test substance is 6-carboxyfluorescein-labeled DNA (16 base pairs) as a fluorescence-modified nucleic acid. The concentration of the test substance in the drug solution is 0.1 mg/ml. The results are illustrated in Fig. 10. In Fig. 10, EP represents a result obtained by performing only electroporation (first step), IP represents a result obtained by performing only iontophoresis (second step), and EP+IP represents a result obtained by performing electroporation (first step) and iontophoresis (second step) in this order.

In Experimental Example 1, the administration conditions of the electric energy in EP (first step) are as follows.
Voltage value: 210 V
Pulse length: 200 ms
Pulse interval: 30 s
Number of pulses: 10

In Experimental Example 1, the administration conditions of the electric energy in the IP (second step) are as follows.
Current density: 2.0 mA/cm²
Current application time: 3 min

The upper part of Fig. 10 is a fluorescence image of a cross section near the porcine cornea, and shows the distribution of a fluorescence-modified nucleic acid as a test substance. In the drawing, a portion having a larger amount of the fluorescence-modified nucleic acid is displayed in white. In addition, a white double-headed arrow in the drawing indicates the range of the corneal epithelium 96. As illustrated in the drawing, the test substance migrates to a deeper portion in the case of sequentially performing the first step and the second step (EP+IP) than in the case of only the first step (EP) and the case of only the second step (IP).

The lower part of Fig. 10 shows a micrograph of a corneal tissue subjected to HE staining. The illustrated results indicate that the corneal tissue has not been degenerated in any of the case of only the first step (EP), the case of only the second step (IP), and the case of using the first step and the second step in combination (EP+IP).

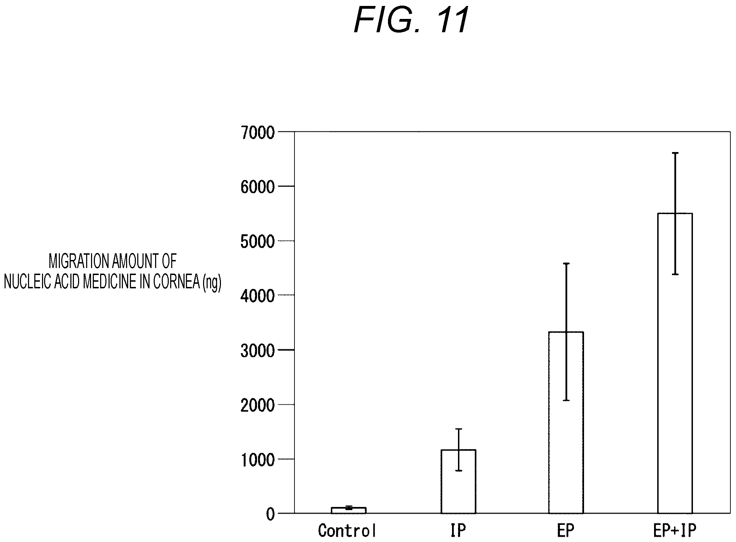

Next, Fig. 11 shows the evaluation results of the migration amount of the test substance of Experimental Example 1. The illustrated results show that the combined use of the first step and the second step (EP+IP) significantly increases the amount of the test substance (nucleic acid medicine) migrated into the cornea 92.

In addition, even in the case of only the first step (EP only), a result similar to the case where the first step and the second step are used in combination (EP+IP) is obtained, and a sufficient migration amount is obtained even in the case of only the first step.

Hereinafter, the results of studying the shapes of the agent reservoir 12 and the first electrode 24 of the intraocular drug administration device 10 will be described.

### (Comparative Example 1)

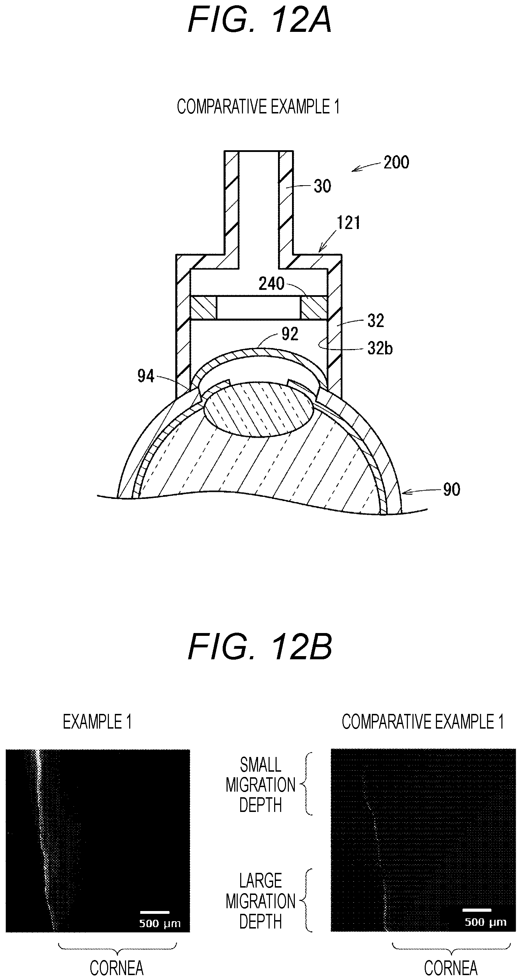

In an administration device 200 of Comparative Example 1, as illustrated in Fig. 12A, the shapes of an agent reservoir 121 and an active electrode 240 are different from the shapes of the agent reservoir 12 and the first electrode 24 according to the first embodiment of Fig. 2A. The agent reservoir 121 has a linearly extending cylindrical shape in which the diameter in the vicinity of the eyeball 90 is set to the same value as the diameter of the cornea 92. The active electrode 240 is a circular ring-shaped electrode, and protrudes short inward from the inner side surface 32b of the outer cylinder portion 32. The active electrode 240 is disposed away from the cornea 92. Note that the active electrode 240 does not cover the cornea 92 and does not have curvature along the surface of the cornea 92.

In Comparative Example 1, the first step and the second step are performed using the active electrode 240 illustrated in Fig. 12A, and the migration depth of the test substance (fluorescence-modified nucleic acid) is evaluated by the fluorescence image. The results are shown in Fig. 12B. The results shown in the drawing show that there are large and small migration depths of the test substance at the corneal surface. Therefore, the active electrode 240 of Comparative Example 1 has been not able to uniformly migrate the agent to the corneal tissue.

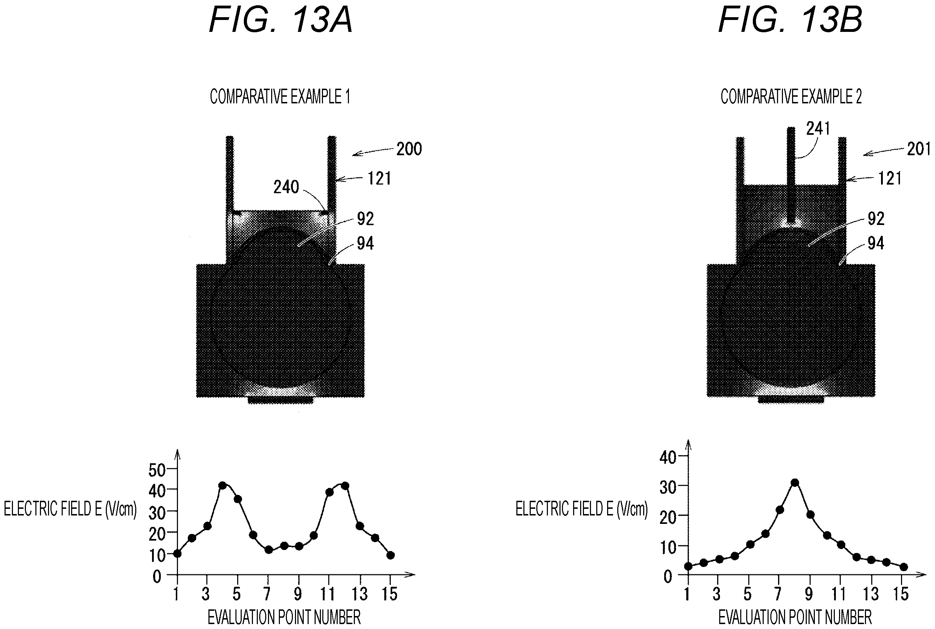

The result of Comparative Example 1 is considered to be generated because there is a region where the formation of the local transport region 100 is partially insufficient due to uneven electric field irradiated to the cornea 92 by the first step (electroporation). Therefore, as illustrated in Fig. 13A, the electric field distribution irradiated to the cornea 92 by the active electrode 240 of Comparative Example 1 has been obtained by simulation.

The upper diagram in Fig. 13A shows that a stronger electric field acts as the color approaches white in shading. As illustrated in the drawing, the electric field generated from the active electrode 240 of Comparative Example 1 is weak at the peripheral edge and the central portion of the cornea 92, and a strong electric field acts at an intermediate annular portion therebetween.

The lower graph in Fig. 13A illustrates a result of the electric field irradiated to the cornea 92 in the upper diagram in Fig. 13A for the evaluation points provided at each of 15 divided positions from left to right along the cross section in the diagram. As illustrated in the graph, the electric field is weak at the peripheral edge and the central portion of the cornea 92, and the electric field is strong at the intermediate portion. The result of Comparative Example 1 shows that the migration depth of the nucleic acid medicine increases in a portion where a strong electric field acts, and the migration depth of the nucleic acid medicine decreases in a portion where a weak electric field acts. In the administration device 200, the intensity of the electric field varies on the surface of the cornea 92, indicating that the range in which the agent 45 moves deep is limited. Since the intensity of the electric field is restricted from the viewpoint of damage to the cornea 92, the migration amount of the agent 45 is limited in the administration device 200.

### (Comparative Example 2)

Comparative Example 2 illustrated in Fig. 13B shows the result of calculating the electric field distribution of the administration device 201 of Jinsong Hao et al. As illustrated in the drawing, an administration device 201 of Comparative Example 2 has an active electrode 241 protruding in a rod shape in the vicinity of the central axis of the cylindrical agent reservoir 121. The active electrode 241 extends in the axial direction of the agent reservoir 121 and is disposed such that its tip faces the center of the cornea 92. As illustrated in the lower graph of Fig. 13B, the electric field is concentrated in the vicinity of the center (evaluation point number 8) of the cornea 92 in the vicinity of the active electrode 241, and is weak in the peripheral portion of the cornea 92. The result of Comparative Example 2 indicates that a uniform electric field cannot be applied to the entire cornea.

### (Comparative Example 3)

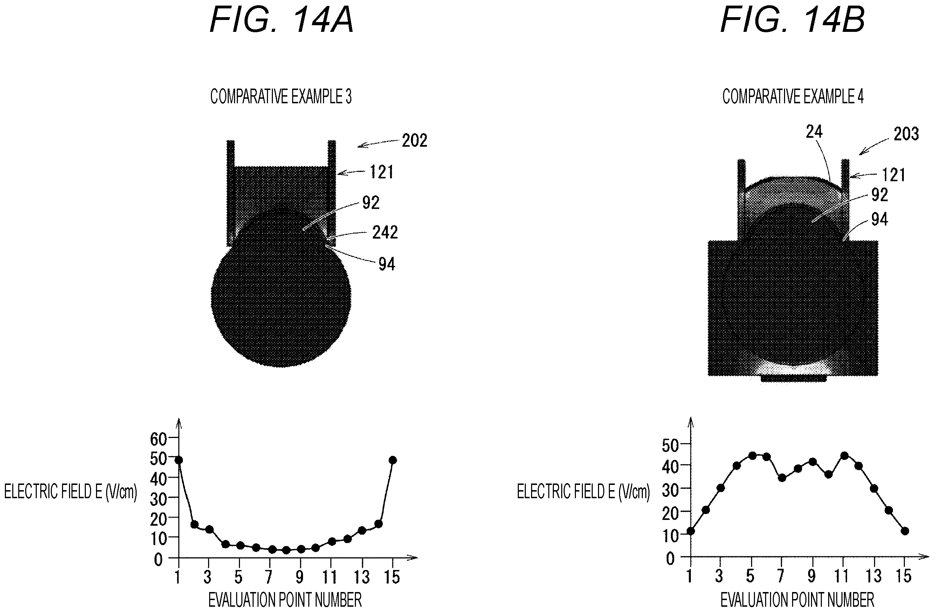

Comparative Example 3 illustrated in Fig. 14A shows a result of calculating the electric field distribution of the administration device 202 described in U.S. Patent No. 8838229. As illustrated in the drawing, the administration device 202 of Comparative Example 3 includes an agent reservoir 121 and a cylindrical active electrode 242. The active electrode 242 of Comparative Example 3 is disposed at a position in contact with the surface of the cornea 92. As illustrated in the drawing, in the administration device 202 of Comparative Example 3, a strong electric field is applied to the peripheral portion of the cornea 92.

As illustrated in the lower diagram of Fig. 14A, in the administration device 202, the highest electric field is applied at the evaluation point numbers 1 and 15 in the vicinity of the corneal limbus 94. In addition, the illustrated result indicates that a sufficient electric field is not applied in the range of the evaluation point number 2 to 14 in the administration device 202.

### (Comparative Example 4)

Comparative Example 4 illustrated in Fig. 14B shows a result of calculating the electric field distribution for an administration device 203 in which the first electrode 24 is combined with the agent reservoir 121 equal to the diameter of the cornea 92. As illustrated in the drawing, in the administration device 203 of Comparative Example 4, by covering the cornea 92 and including the first electrode 24 curved along the surface of the cornea 92, it has been confirmed that a uniform electric field can be applied over a wider range as compared with Comparative Examples 1 to 3.

That is, as illustrated in the lower graph of Fig. 14B, a substantially uniform electric field is applied in the range of the evaluation point number 4 to 12. However, in the evaluation point numbers 1 to 3 and 13 to 15 which are the peripheral edges of the cornea 92, the intensity of the electric field decreases, indicating that there is room for further improvement.

### (Example 1)

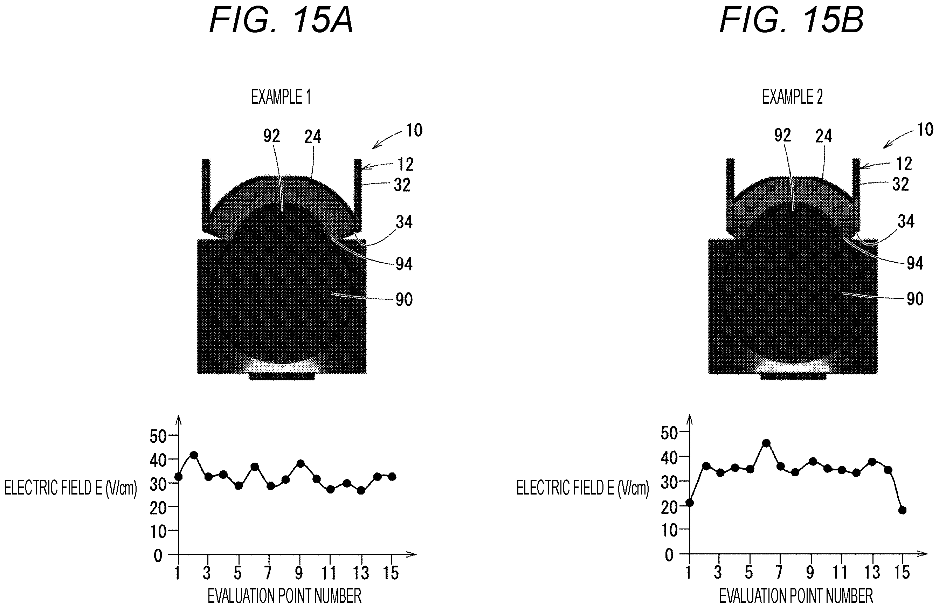

Example 1 illustrated in Fig. 15A illustrates a result of calculating the distribution of the electric field applied to the cornea 92 by the intraocular drug administration device 10 according to the first embodiment. The intraocular drug administration device 10 includes an agent reservoir 12 having a maximum diameter larger than the diameter of the cornea 92 as illustrated in Fig. 2A, and a first electrode 24 that covers the cornea 92 and is curved along the cornea 92. In the present embodiment, the inner diameter of the outer cylinder portion 32 of the agent reservoir 12 is set to a value 3.8 mm away outward from the corneal limbus 94. The curved shape of the first electrode 24 is a spherical shape having a curvature radius of 12.7 mm. The shortest distance between the first electrode 24 and the cornea 92 is set to 4 mm. The voltage applied between the first electrode 24 and the second electrode 14 in the first step is 70 V.

In Example 1 illustrated in the upper diagram of Fig. 15A, a substantially uniform electric field is applied to the entire cornea 92. As illustrated in the lower graph of Fig. 15A, a substantially uniform electric field is applied in the range of the evaluation point numbers 1 to 15. This result indicates that the intraocular drug administration device 10 of the present embodiment can efficiently migrate much agent 45 into the cornea 92.

### (Example 2)

Example 2 illustrated in Fig. 15B is a modification in which the diameter of the outer cylinder portion 32 of the agent reservoir 12 of Example 1 is further reduced. The inner diameter of the outer cylinder portion 32 of Example 2 is set to a value 1.8 mm away outward from the corneal limbus 94. The other conditions are the same as in Example 1.

As illustrated in the upper diagram of Fig. 15B, in Example 2, a uniform electric field is applied in a portion other than the corneal limbus 94. As illustrated in the lower graph of Fig. 15B, the strength of the electric field decreases in evaluation point numbers 1 and 15, while a substantially uniform electric field is applied in evaluation point numbers 2 to 14. The results of this example show that a uniform electric field can be applied over most of the cornea 92, and the agent 45 can be efficiently migrated into the cornea 92. The corneal limbus 94 is a site where many stem cells regenerating the cells 97 of the cornea 92 exist. Therefore, the present embodiment is effective in avoiding electrical stimulation to the corneal limbus 94.

### (Example 3)

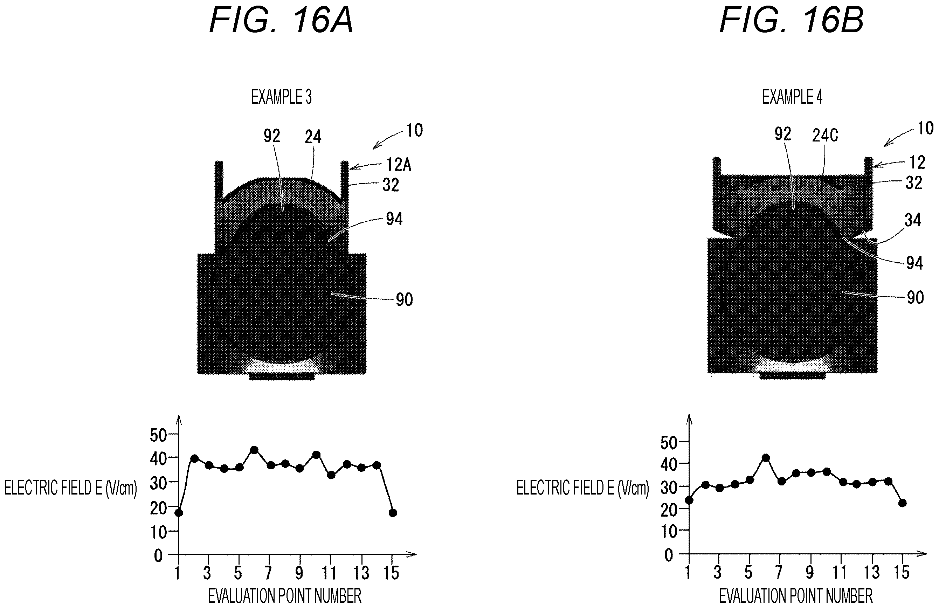

Example 3 illustrated in Fig. 16A has an agent reservoir 12A. The agent reservoir 12A is similar to the third modification (see Fig. 7A) in which the reduced diameter portion 34 of the agent reservoir 12 of Example 1 is not provided. In the agent reservoir 12A of Example 3, the lower end of the outer cylinder portion 32 abuts on the eyeball 90. The inner diameter of the outer cylinder portion 32 is set to a value 1.8 mm away outward from the corneal limbus 94. The other conditions are the same as in Example 1.

As illustrated in the upper diagram of Fig. 16A, in Example 3, a uniform electric field is applied in a portion other than the corneal limbus 94. As illustrated in the lower graph of Fig. 16A, the strength of the electric field decreases in evaluation point numbers 1 and 15, while a substantially uniform electric field is applied in evaluation point numbers 2 to 14. The results of this example show that a uniform electric field can be applied over most of the cornea 92, and the agent 45 can be efficiently migrated into the cornea 92. In addition, the present embodiment shows a meaningful result in the case of avoiding electrical stimulation to the corneal limbus 94.

### (Example 4)

Example 4 illustrated in Fig. 16B has a first electrode 24C. The first electrode 24C is a modification (see Example 4 and Fig. 7B) in which the diameter of the first electrode 24 of Example 1 is reduced. This example is similar to Example 1 except that the diameter of the first electrode 24B is reduced to the same value as the diameter of the cornea 92.

As illustrated in the lower graph of Fig. 16B, a substantially uniform electric field is applied over the range of the evaluation point numbers 1 to 15. This result indicates that the agent 45 can be efficiently migrated to the inside of the cornea 92 also in the present embodiment.

### (Example 5 and Example 6)

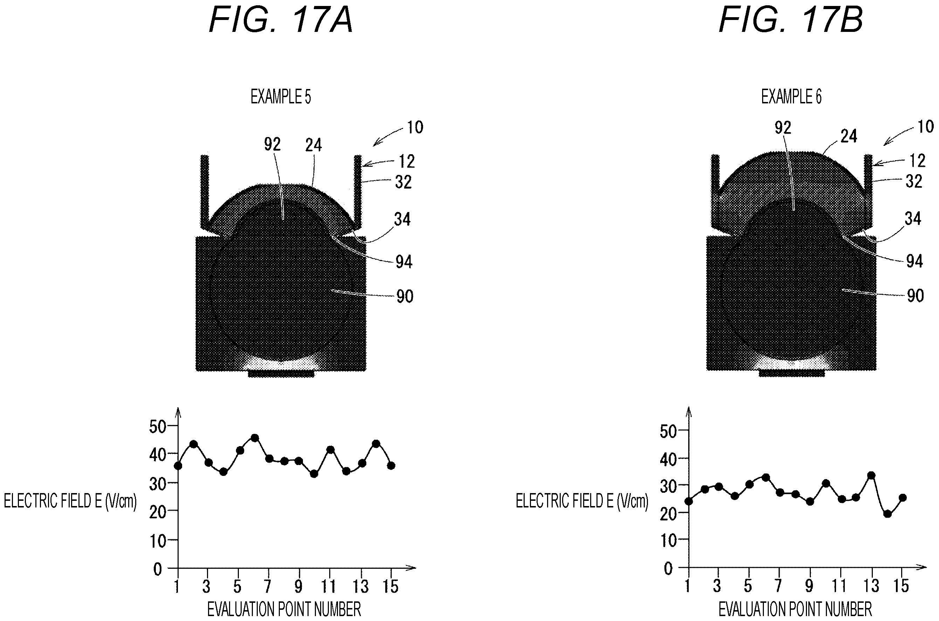

Example 5 illustrated in Fig. 17A and Example 6 illustrated in Fig. 17B illustrate results of examination in consideration of individual differences of the eyeball 90 and the cornea 92. In Examples 5 and 6, the curvature radius of the first electrode 24 is fixed to 12.7 mm, and the distance between the first electrode 24 and the cornea 92 is changed from 4 mm in Example 1. That is, in Example 5, the shortest distance between the first electrode 24 and the cornea 92 is 2 mm, and in Example 6, the shortest distance between the first electrode 24 and the cornea 92 is 7 mm. The other conditions are the same as those in Example 1.

Fig. 17A shows the calculation result of the electric field distribution of Example 5. As illustrated in the graph of Fig. 17A, it has been confirmed that an electric field having a substantially uniform distribution has been applied at the evaluation point numbers 1 to 15 also in Example 5.

Fig. 17B shows the calculation result of the electric field distribution of Example 6. As illustrated in the graph of Fig. 17B, it has been confirmed that an electric field having a substantially uniform distribution has been applied also in Example 6 at the evaluation point numbers 1 to 15.

As in Examples 5 and 6 described above, the intraocular drug administration device 10 according to the first embodiment can apply a uniform electric field even in a case where the shortest distance between the first electrode 24 and the cornea 92 slightly varies as compared with the case of Example 1 due to individual differences or the like.

### (Second Embodiment)

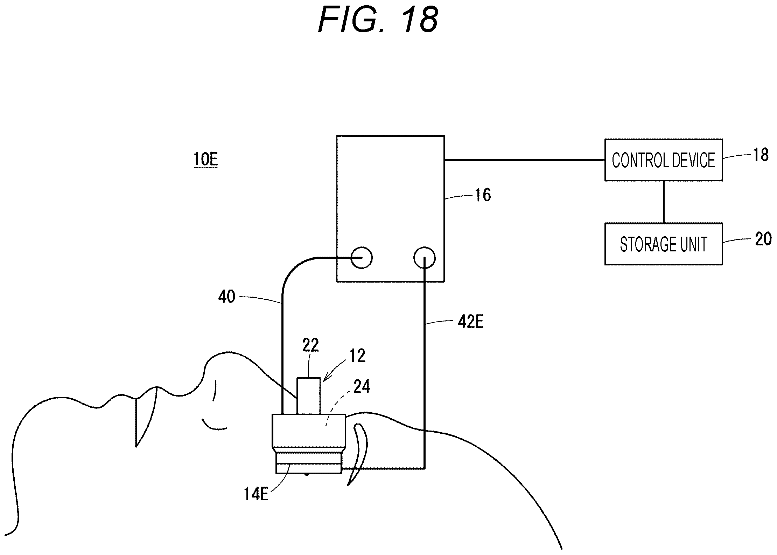

As illustrated in Fig. 18, an intraocular drug administration device 10E according to the present embodiment is different from the intraocular drug administration device 10 of Fig. 1 in the agent reservoir 12 to which a second electrode 14E is attached. Note that, in the configuration of the intraocular drug administration device 10E, the same reference numerals are assigned to the same configurations as those of the intraocular drug administration device 10 in Fig. 1, and a detailed description thereof will be omitted.

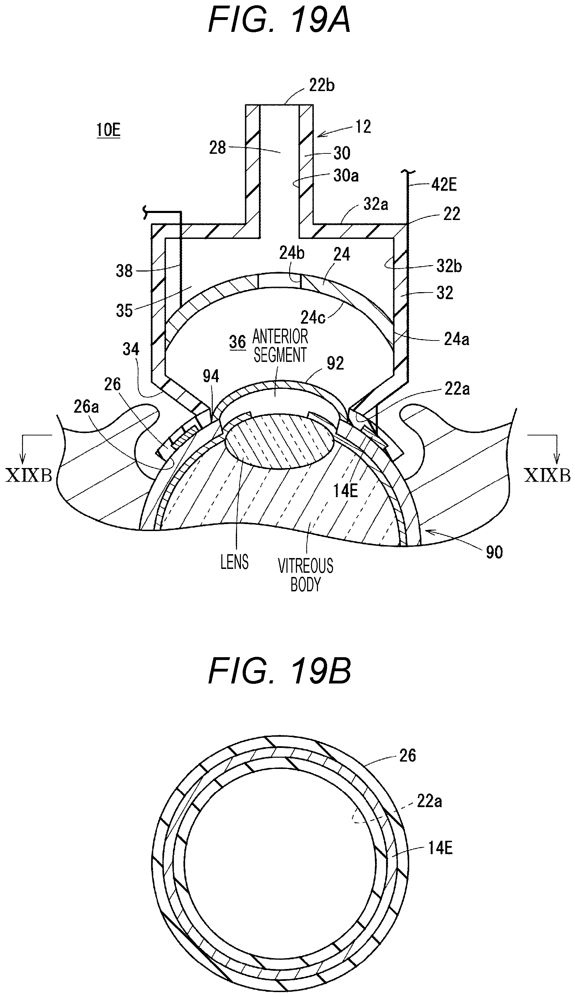

As illustrated in Fig. 19A, the agent reservoir 12 of the present embodiment has a second electrode 14E. The second electrode 14E is attached to the seal member 26. The seal member 26 has a circular contact surface 26a (contact portion) that contacts the eyeball 90. The second electrode 14E is formed on the contact surface 26a and can contact the eyeball 90.

As illustrated in Fig. 19B, the second electrode 14E extends annularly along the seal member 26. The second electrode 14E extends so as to surround the outer periphery of the lower end portion 22a of the agent reservoir 12. The second electrode 14E has an inner diameter larger than that of the cornea 92, and the second electrode 14E abuts on the eyeball 90 outside the cornea 92. More preferably, the second electrode 14E can be formed in an annular shape having a diameter abutting on the conjunctiva.

The annularly extending second electrode 14E can prevent local concentration of an electric field when a voltage is applied, and can generate a uniform electric field over the entire region of the cornea 92. Note that the second electrode 14E is not limited to an annular shape continuously connected in the circumferential direction as long as a uniform electric field can be generated over the entire region of the cornea 92. For example, a plurality of the second electrodes 14E may be arranged to be dispersed in the circumferential direction along the annular region. In addition, the second electrode 14E may be configured as a mesh-like pattern along an annular region.

As illustrated in Fig. 19A, a wiring 42E is connected to the second electrode 14E. The wiring 42E of the agent reservoir 12 is disposed along the surface of the main body portion 22. The wiring 42E penetrates the seal member 26 and is electrically connected to the second electrode 14E.

The intraocular drug administration device 10E of the present embodiment as described above can administer the agent 45 into the eye by applying a high-voltage pulse between the first electrode 24 and the second electrode 14E and supplying a constant current as necessary. In the intraocular drug administration device 10E, since the second electrode 14E abuts on the same eyeball 90 as the first electrode 24, the energization range at the time of administration can be limited to only the anterior segment. In the intraocular drug administration device 10E of the present embodiment, since energization conditions on the organism side such as a resistance value are stabilized, the agent 45 can be administered into the eye more effectively.

### (Modification of Second Embodiment)

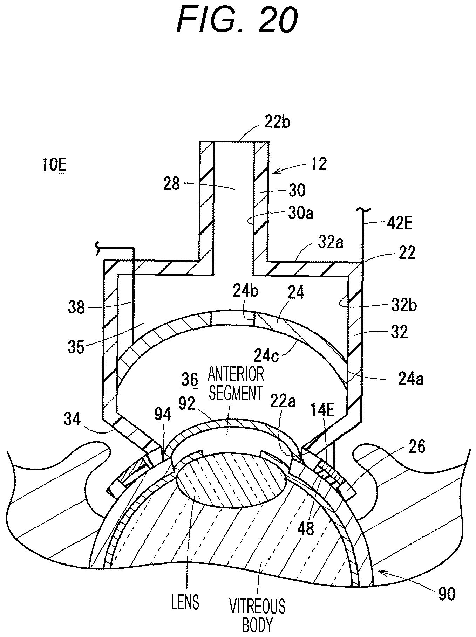

As illustrated in Fig. 20, the present modification is different from Fig. 19A in the arrangement of the second electrode 14E. In the example of Fig. 19A, the second electrode 14E is exposed on the contact surface 26a of the seal member 26, and the second electrode 14E is configured to directly contact the eyeball 90, but the present embodiment is not limited thereto. In the present modification, the second electrode 14E is disposed inside the seal member 26. In addition, the second electrode 14E is covered with a coating layer 48 having conductivity. That is, in the present modification, the second electrode 14E is not exposed and abuts on the eyeball 90 via the coating layer 48.

The coating layer 48 is, for example, a flexible and conductive material, and is, for example, a conductive urethane foam or a hydrophilic polymer such as polyacrylic acid. In the intraocular drug administration device 10E to which the present modification is applied, since the second electrode 14E does not directly hit the conjunctiva, damage to the tissue can be further reduced.

### (Example 7)

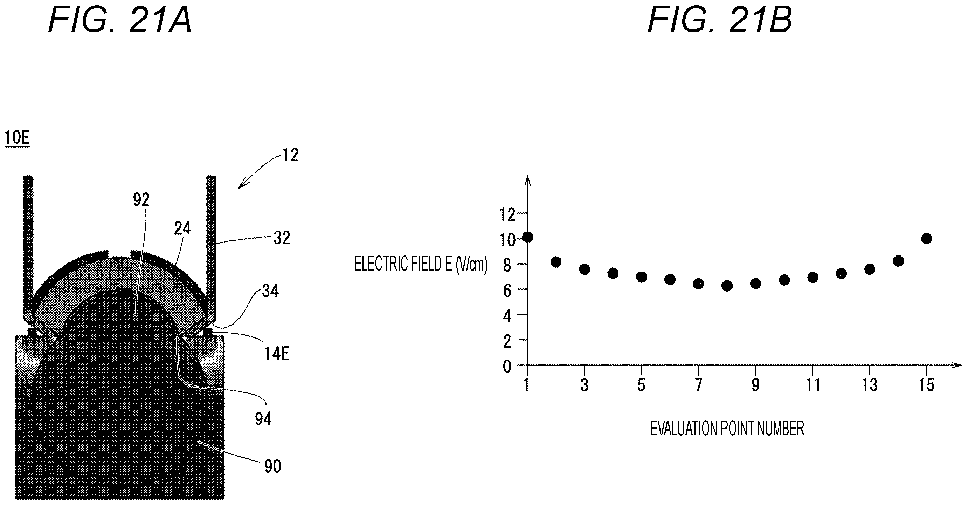

In the present example, the distribution of the electric field of the cornea 92 and its periphery is calculated by the intraocular drug administration device 10E according to the second embodiment. As illustrated in Fig. 21A, in Example 7, a substantially uniform electric field is applied to the entire cornea 92. In Example 7, it can be seen that the portion to which the electric field is applied is limited to the eye surface. This result indicates that the intraocular drug administration device 10E can suppress damage to the posterior ocular tissue.

In addition, as illustrated in Fig. 21B, the electric field distribution along the surface of the cornea 92 shows a tendency that the electric field intensity increases on the outer peripheral portion side of the cornea 92, but the difference between the maximum value and the minimum value of the electric field intensity is only 40%, and is substantially uniform. Therefore, the intraocular drug administration device 10E of the present embodiment can efficiently migrate the drug into the tissue using the wide area of the surface of the tissue of the eyeball 90, and can increase the amount of the drug migrated to the tissue of the eyeball 90.

Note that the present invention is not limited to the disclosure, and various configurations can be adopted without departing from the gist of the present invention.

## Claims

1. An intraocular drug administration device comprising:
an agent reservoir that is attached to an eyeball of an organism, and has an outer cylinder portion holding an agent in a state of being in contact with the eyeball;
a first electrode that is provided in the outer cylinder portion so as to be separated from the eyeball and cover at least a part of the eyeball, and has a curved shape so as to be recessed in a direction away from the eyeball;
a second electrode that is attached to an organism site energizable between the second electrode and the first electrode; and
a power supply that supplies a current between the first electrode and the second electrode.

2. The intraocular drug administration device according to claim 1, wherein the first electrode has a curved surface shape.

3. The intraocular drug administration device according to claim 2, wherein the curved surface shape of the first electrode is a shape along a spherical surface or an elliptical surface.

4. The intraocular drug administration device according to claim 1, wherein
the first electrode has a curved shape with a curvature radius of 6 mm to 20 mm.

5. The intraocular drug administration device according to claim 1, wherein
the second electrode is attached to the same eyeball as the eyeball to which the agent reservoir is attached, and
a lower end portion of the outer cylinder portion has a circular contact portion surrounding the corneal limbus of the eyeball, and the second electrode is disposed continuously or dispersedly so as to surround a cornea along an annular region surrounding the contact portion.

6. The intraocular drug administration device according to claim 5, wherein the second electrode is attached to a conjunctiva.

7. The intraocular drug administration device according to claim 5, wherein the second electrode is electrically connected to the eyeball via a conductive covering layer.

8. The intraocular drug administration device according to claim 1, wherein the second electrode is attached to a forehead of an organism.

9. The intraocular drug administration device according to claim 1, wherein
an inner surface of the outer cylinder portion of the agent reservoir has a radial clearance outside a corneal limbus of the eyeball.

10. The intraocular drug administration device according to claim 1, wherein the outer cylinder portion of the agent reservoir has an inner diameter larger than a diameter of a cornea of the eyeball.

11. The intraocular drug administration device according to claim 9, wherein the outer cylinder portion of the agent reservoir has a reduced diameter portion whose inner diameter decreases as approaching the eyeball between the first electrode and the eyeball.

12. The intraocular drug administration device according to claim 9, wherein the first electrode has an outer diameter equal to or larger than a diameter of the corneal limbus.

13. The intraocular drug administration device according to claim 12, wherein the first electrode has a diameter of 9.1 mm to 30 mm.

14. The intraocular drug administration device according to claim 12, wherein an outer diameter of the first electrode is smaller than an inner diameter of an upper end of the reduced diameter portion, and has a size equal to or larger than an inner diameter of a lower end of the reduced diameter portion.

15. The intraocular drug administration device according to claim 1, wherein the first electrode has a through-hole through which a fluid passes.

16. The intraocular drug administration device according to claim 15, wherein the through-hole is formed in a central portion of the first electrode.

17. The intraocular drug administration device according to claim 1, wherein the agent reservoir has a seal member made of an elastic member at a lower end portion.

18. The intraocular drug administration device according to any one of claims 1 to 17, further comprising:
a control device that controls the power supply; and
a storage unit that stores a command to be executed by the control device, wherein
when the command is executed, the control device
performs a first step of applying a high-voltage pulse between the first electrode and the second electrode through the power supply.

19. The intraocular drug administration device according to claim 18, wherein
the control device
performs a second step of causing a constant current having a smaller current value than the high-voltage pulse to flow between the first electrode and the second electrode through the power supply for a predetermined time after the first step.

20. An agent reservoir, comprising:
an outer cylinder portion that is attached to an eyeball of an organism and holds an agent in a state of being in contact with the eyeball; and
a first electrode that is provided in the outer cylinder portion to be separated from the eyeball to cover at least a part of the eyeball, and is curved to be recessed in a direction away from the eyeball.

21. A method for operating an intraocular drug administration device including an agent reservoir that is attached to an eyeball of an organism and has an outer cylinder portion holding an agent in a state of being in contact with the eyeball, a first electrode that is provided in the outer cylinder portion so as to be separated from the eyeball and cover at least a part of the eyeball, and is curved so as to be recessed in a direction away from the eyeball, a second electrode that can be energized between the first electrode and the second electrode, a power supply that supplies a current between the first electrode and the second electrode, a control device that controls the power supply, and a storage unit that stores a command to be executed by the control device, the method comprising:
a first step, performed by the control device, of controlling the power supply to apply a high-voltage pulse between the first electrode and the second electrode a plurality of times.

22. The method for operating the intraocular drug administration device according to claim 21, the method comprising:
a second step of causing a constant current having a smaller current value than the high-voltage pulse to flow between the first electrode and the second electrode through the power supply for a predetermined time after the first step.
